# EUROPEAN PATENT SPECIFICATION

(11) **EP 3 576 613 B1**
(45) Date of publication and mention of the grant of the patent: **11.09.2024**
(21) Application number: 18748771.5
(22) Date of filing: 30.01.2018
(51) Int. Cl.: A61B 5/00, A61B 5/01, A61M 25/10

(54) **CRYOGENIC BALLOON CATHETER SYSTEM WITH SENSOR ASSEMBLY**
KRYOGENES BALLONKATHETERSYSTEM MIT SENSORANORDNUNG
SYSTÈME DE CATHÉTER À BALLONNET CRYOGÉNIQUE AVEC ENSEMBLE CAPTEUR

(30) Priority: 31.01.2017 US 201762452973 P; 04.01.2018 US 201862613722 P
(43) Date of publication of application: 11.12.2019
(73) Proprietor: Boston Scientific Scimed, Inc., Maple Grove, MN 55311 (US)
(72) Inventor: JUNG, JR., Eugene J., San Diego, CA 92130 (US); HARPER, Keegan, Encinitas, CA 92024 (US); ROMAN, Ricardo, Chula Vista, CA 91911 (US)
(74) Representative: Pfenning, Meinig & Partner mbB
(86) International application number: PCT/US2018/016026
(87) International publication number: WO 2018/144483

(56) References cited:
- EP-A1- 3 037 034
- EP-A1- 3 178 431
- US-A1- 2010 087 782
- US-A1- 2011 299 567
- US-A1- 2012 165 803
- US-A1- 2013 165 990
- US-A1- 2014 012 160
- US-A1- 2014 180 077
- US-A1- 2014 357 956
- US-B1- 6 190 355

## Description

### RELATED APPLICATIONS

This application claims priority on U.S. Provisional Application Serial No. 62/452,973, filed on January 31, 2017, and entitled "CRYOGENIC BALLOON CATHETER ASSEMBLY WITH SENSOR ASSEMBLY", and on U.S. Provisional Application Serial No. 62/613,722, filed on January 4, 2018, and entitled "CRYOGENIC BALLOON CATHETER ASSEMBLY WITH SENSOR ASSEMBLY".

### BACKGROUND

Cardiac arrhythmias involve an abnormality in the electrical conduction of the heart and are a leading cause of stroke, heart disease, and sudden cardiac death. Treatment options for patients with arrhythmias include medications, implantable devices, and catheter ablation of cardiac tissue.

Catheter ablation involves delivering ablative energy to tissue inside the heart to block aberrant electrical activity from depolarizing heart muscle cells out of synchrony with the heart's normal conduction pattern. The procedure is performed by positioning the tip of an energy delivery catheter adjacent to diseased or targeted tissue in the heart. The energy delivery component of the system is typically at or near the most distal (furthest from the operator) portion of the catheter, and often at a tip of the device. Various forms of energy are used to ablate diseased heart tissue. These can include radio frequency (RF), cryogenics, ultrasound and laser energy, to name a few. The tip of the catheter is positioned adjacent to diseased tissue, at which time energy is delivered to create tissue necrosis, rendering the ablated tissue incapable of conducting electrical signals. The dose of the energy delivered is a critical factor in increasing the likelihood that the treated tissue is permanently incapable of conduction. At the same time, delicate collateral tissue, such as the esophagus, the bronchus, and the phrenic nerve surrounding the ablation zone can be damaged and can lead to undesired complications. Thus, the operator must finely balance delivering therapeutic levels of energy to achieve intended tissue necrosis while avoiding excessive energy leading to collateral tissue injury.

Atrial fibrillation (AF) is one of the most common arrhythmias treated using catheter ablation. In the earliest stages of the disease, paroxysmal AF, the treatment strategy involves isolating the pulmonary veins from the left atrial chamber. Recently, the use of techniques known as "balloon cryotherapy" catheter procedures to treat AF have increased. In part, this stems from the balloon cryotherapy's ease of use, shorter procedure times and improved patient outcomes. Despite these advantages, there remains needed improvement to further improve patient outcomes and to better facilitate real-time physiological monitoring of tissue to optimally titrate energy to perform both reversible "ice mapping" and permanent tissue ablation.

There remains an unmet need for an expansible tissue ablation device that is capable of providing effective therapeutic ablative energy and reliable physiological monitoring to improve delivery of ablative energy to achieve improved therapeutic outcomes. There is also a need to reduce excessive energy delivery that causes collateral tissue injury. The device should ideally control the amount of therapeutic energy based on real-time physiological monitoring to control the amount of ablative energy delivered to the tissue at a specified target location. This should include a manner of modulating ablative energy output based on real-time interrogation of tissue parameters such as temperature, device contact force, local blood pressure, etc.

Moreover, there is an unmet need for a device which can seamlessly integrate physiological monitoring sensors and ablative elements without compromising the function of the device in administering therapeutic energy or enabling device conversion between a collapsed state and an expanded state.

Further, there is a need for a cryoablation balloon catheter that treats atrial fibrillation and other arrhythmias, which can sense tissue parameters in real-time before, during and after ablation. Currently, the "mapping" function in cryoablation catheters is often handled by separate accessory devices. Adding conventional sensors to a balloon can add undesirable bulk to the balloon, which would necessitate a larger delivery sheath to introduce into and retract from the body of the patient.

There have been several attempts to attach sensors or mapping electrodes to balloons, all with limited success. Same disadvantages to earlier solutions include added bulk or balloon profile, a tendency for the electrodes to peel off a balloon during expansion and contraction, and difficulty routing wires to connect to the electrodes, to name a few.

US 2014/012160 A1 discusses an apparatus for medical diagnosis and/or treatment. The apparatus includes a flexible substrate forming an inflatable body and a plurality of force sensing elements disposed on the flexible substrate. The plurality of force sensing elements are disposed about the inflatable body such that the force sensing elements are disposed at areas of minimal curvature of the inflatable body in a deflated state.

US 2014/357956 A1 discusses an ablation catheter comprising: an expandable membrane and a plurality of ablation electrodes secured to the exterior of the expandable membrane; an imaging member disposed within the expandable membrane; a diffuse reflector secured to at least a proximal portion of the expandable membrane; and a light source disposed within the expandable member and positioned to direct light towards the diffuse reflector such that diffuse reflection of the light is directed towards a field of view of the imaging member.

EP 3 178 431 A1 discusses an expandable structure and one or more sets of electrodes disposed on the surface of the expandable structure. Each one of the sets comprises an ablating electrode, and at least one sensing electrode that is electrically isolated from the ablating electrode. The sensing electrode is contained within the ablating electrode.

EP 3 037 034 A1 discusses cardiac ablation which is carried out by introducing a catheter into the left atrium, extending a lasso guide through the lumen of the catheter to engage the wall of a pulmonary vein, and deploying a balloon over the lasso guide. The balloon has an electrode assembly disposed its exterior. The electrode assembly includes a plurality of ablation electrodes circumferentially arranged about the longitudinal axis of the catheter. The inflated balloon is positioned against the pulmonary vein ostium, so that the ablation electrodes are in galvanic contact with the pulmonary vein, and electrical energy is conducted through the ablation electrodes to produce a circumferential lesion that circumscribes the pulmonary vein.

### SUMMARY OF THE INVENTION

The invention is set out in the appended set of claims.

### Summary of the Disclosure

The present disclosure is directed toward an intravascular catheter system for treating a condition in a body. In one example, the intravascular catheter system includes a catheter shaft, a first inflatable balloon and a plurality of electrodes. The catheter shaft has a shaft distal end that is selectively positioned within the body. The first inflatable balloon is positioned near the distal end of the catheter shaft. The first inflatable balloon is configured to move between an inflated state and a substantially deflated state. In the inflated state, the first inflatable balloon has a maximum circumference. The plurality of electrodes are attached to the first inflatable balloon. The plurality of electrodes can sense a physiological parameter within the body. Further, the plurality of electrodes can be positioned away from the maximum circumference of the first inflatable balloon so that none of the electrodes are positioned on the maximum circumference of the first inflatable balloon.

In certain examples, the first inflatable balloon has an inner surface and an opposed outer surface. In some such embodiments, the electrodes are positioned on the inner surface of the first inflatable balloon.

In another example, the first inflatable balloon has an inner surface and an opposed outer surface. In this embodiment, the electrodes are positioned on the outer surface of the first inflatable balloon.

In various examples, the intravascular catheter system can also include one or more flex circuits that are secured to the first inflatable balloon. In certain such examples, the electrodes are coupled to the first inflatable balloon via the one or more flex circuits. In various examples, the one or more flex circuits can be positioned away from the maximum circumference of the first inflatable balloon. In some examples, each of the flex circuits can be secured to the first inflatable balloon distal to the maximum circumference.

In certain examples, at least two electrodes are positioned on each of the one or more flex circuits. In some such examples, two of the electrodes on each flex circuit form a thermocouple.

In various examples, at least eight flex circuits are positioned on the first inflatable balloon. Alternatively, at least twelve flex circuits are positioned on the first inflatable balloon.

In some examples, the inflatable balloon includes a plurality of spines when the first inflatable balloon is in the substantially deflated state. In certain examples, two flex circuits are positioned between two adjacent spines. In some examples, two adjacent two flex circuits substantially face one another when the first inflatable balloon is in the substantially deflated state.

In another example, the intravascular catheter system can also include a second inflatable balloon that is positioned within the first inflatable balloon. In some such examples, the second inflatable balloon has an outer surface and an opposed inner surface, and the plurality of electrodes are positioned on the outer surface of the second inflatable balloon.

In various example, the intravascular catheter system also includes a guidewire lumen and a guidewire that is positioned at least partially within the guidewire lumen. The first inflatable balloon can be attached to the guidewire lumen. The intravascular catheter system can also include a controller and a plurality of conductors. In certain examples, each conductor carries an electrical signal between at least one of the electrodes and the controller. The electrical signal can be based on the physiological parameter.

In some examples, the guidewire lumen has a lumen distal end, and each conductor is routed from at least one of the electrodes to the controller via the lumen distal end of the guidewire lumen.

In certain examples, the intravascular catheter system can also include a pair of reference electrodes that are positioned away from the first inflatable balloon. In these examples, the pair of reference electrodes can form a thermocouple that senses a temperature of a portion of the body. The reference electrodes can generate a reference sensor output. In various examples, two of the plurality of electrodes generate a sensor output that is compared to the reference sensor output to determine a temperature of a portion of the body.

In another example, the intravascular catheter system can include a catheter shaft, a first inflatable balloon, a flex circuit and a pair of electrodes. The catheter shaft can have a shaft distal end that is selectively positioned within the body. The first inflatable balloon can be positioned near the distal end of the catheter shaft. The first inflatable balloon can be configured to move between an inflated state and a substantially deflated state. The flex circuit can be attached to the first inflatable balloon. The pair of electrodes can be secured to the flex circuit. The pair of electrodes can sense a physiological parameter within the body.

In a further example, the intravascular catheter system can include a catheter shaft, a first inflatable balloon and a plurality of electrodes. The catheter shaft can have a shaft distal end that is selectively positioned within the body. The first inflatable balloon can be positioned near the distal end of the catheter shaft. The first inflatable balloon can be configured to move between an inflated state and a substantially deflated state. The first inflatable balloon has an inner surface and an opposed outer surface. The plurality of electrodes each senses a physiological parameter within the body. In certain examples, the plurality of electrodes are attached to the inner surface of the first inflatable balloon.

In yet another example, the intravascular catheter system includes a catheter shaft, a first inflatable balloon, a plurality of electrodes and two flex circuits. The catheter shaft can have a shaft distal end that is selectively positioned within the body. The first inflatable balloon can be positioned near the distal end of the catheter shaft. The first inflatable balloon can be configured to move between an inflated state and a substantially deflated state. The plurality of electrodes sense one or more physiological parameters within the body. The two flex circuits couple the electrodes to the first inflatable balloon. In some examples, the two flex circuits can each be attached to the first inflatable balloon. In various examples, the two flex circuits substantially face one another when the first inflatable balloon is in the substantially deflated state.

### BRIEF DESCRIPTION OF THE DRAWINGS

The novel features of this invention, as well as the invention itself, both as to its structure and its operation, will be best understood from the accompanying drawings, taken in conjunction with the accompanying description, in which similar reference characters refer to similar parts, and in which:
Figure 1 is a schematic side view illustration of a patient and one embodiment of a cryogenic balloon catheter system having features of the present invention;
Figure 2A is a simplified side view of a portion of a patient and a portion of one embodiment of the cryogenic balloon catheter system including a balloon catheter;
Figure 2B is a cross-sectional view of the balloon catheter taken on line 2B-2B in Figure 2A;
Figure 2C is a simplified side view of a portion of a patient and a portion of another embodiment of the cryogenic balloon catheter system;
Figure 3A is a cross-sectional view of one embodiment of a portion of the cryogenic balloon catheter system;
Figure 3B is a cross-sectional view of another embodiment of a portion of the cryogenic balloon catheter system;
Figure 4 is a cross-sectional view of the portion of the cryogenic balloon catheter system taken on line 4-4 in Figure 3A;
Figure 5A is a perspective view of a portion of another embodiment of the cryogenic balloon catheter system including an inflatable balloon shown in an inflated state, and a portion of a sensor assembly;
Figure 5B is an end view of a portion of the cryogenic balloon catheter system illustrated in Figure 5A, with the inflatable balloon shown in the inflated state; and
Figure 5C is a perspective view of a portion of the cryogenic balloon catheter system illustrated in Figure 5A, with the inflatable balloon shown in a substantially deflated state.

### DESCRIPTION

Embodiments of the present invention are described herein in the context of a cryogenic balloon catheter system (also hereinafter sometimes referred to as an "intravascular catheter system"). Those of ordinary skill in the art will realize that the following detailed description of the present invention is illustrative only and is not intended to be in any way limiting. Other embodiments of the present invention will readily suggest themselves to such skilled persons having the benefit of this disclosure. Reference will now be made in detail to implementations of the present invention as illustrated in the accompanying drawings.

In the interest of clarity, not all of the routine features of the implementations described herein are shown and described. It will, of course, be appreciated that in the development of any such actual implementation, numerous implementation-specific decisions must be made in order to achieve the developer's specific goals, such as compliance with application-related and business-related constraints, and that these specific goals will vary from one implementation to another and from one developer to another. Moreover, it will be appreciated that such a development effort might be complex and time-consuming, but would nevertheless be a routine undertaking of engineering for those of ordinary skill in the art having the benefit of this disclosure.

Although the disclosure provided herein focuses mainly on cryogenics, it is understood that various other forms of energy can be used to ablate diseased heart tissue. These can include radio frequency (RF), ultrasound and laser energy, as non-exclusive examples.

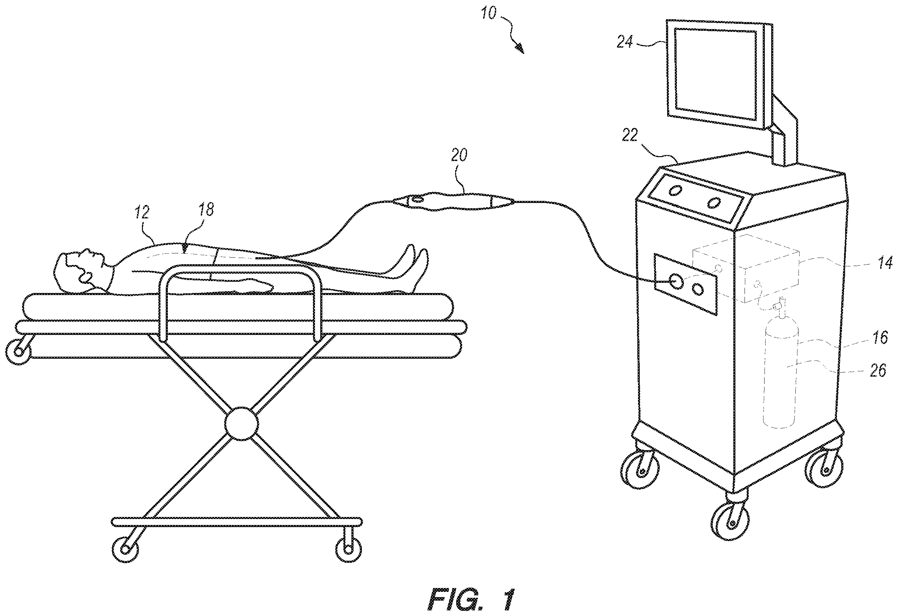

Figure 1 is a schematic side view illustration of one embodiment of a medical device 10 for use with a patient 12, which can be a human being or an animal. Although the specific medical device 10 shown and described herein pertains to and refers to a cryogenic balloon catheter system 10, it is understood and appreciated that other types of medical devices 10 can equally benefit by the teachings provided herein. The design of the cryogenic balloon catheter system 10 can be varied. In certain embodiments such as the embodiment illustrated in Figure 1, the cryogenic balloon catheter system 10 can include one or more of a control system 14, a fluid source 16, a balloon catheter 18, a handle assembly 20, a control console 22 and a graphical display 24. It is understood that although Figure 1 illustrates the structures of the cryogenic balloon catheter system 10 in a particular position, sequence and/or order, these structures can be located in any suitably different position, sequence and/or order than that illustrated in Figure 1.

In various embodiments, the control system 14 can control release and/or retrieval of a cryogenic fluid 26 to and/or from the balloon catheter 18. In various embodiments, the control system 14 can control activation and/or deactivation of one or more other processes of the balloon catheter 18. Additionally, or in the alternative, the control system 14 can receive electrical signals, including data and/or other information (hereinafter sometimes referred to as "sensor output") from various structures within the cryogenic balloon catheter system 10. In some embodiments, the control system 14 can assimilate and/or integrate the sensor output, and/or any other data or information received from any structure within the cryogenic balloon catheter system 10. Additionally, or in the alternative, the control system 14 can control positioning of portions of the balloon catheter 18 within the body of the patient 12, and/or can control any other suitable functions of the balloon catheter 18.

The fluid source 16 contains the cryogenic fluid 26, which is delivered to the balloon catheter 18 with or without input from the control system 14 during a cryoablation procedure. The type of cryogenic fluid 26 that is used during the cryoablation procedure can vary. In one non-exclusive embodiment, the cryogenic fluid 26 can include liquid nitrous oxide. However, any other suitable cryogenic fluid 26 can be used.

The balloon catheter 18 is inserted into the body of the patient 12. In one embodiment, the balloon catheter 18 can be positioned within the body of the patient 12 using the control system 14. Alternatively, the balloon catheter 18 can be manually positioned within the body of the patient 12 by a health care professional (also sometimes referred to herein as an "operator"). In certain embodiments, the balloon catheter 18 is positioned within the body of the patient 12 utilizing the sensor output from the balloon catheter 18. In various embodiments, the sensor output is received by the control system 14, which then can provide the operator with information regarding the positioning of the balloon catheter 18. Based at least partially on the sensor output feedback received by the control system 14, the operator can adjust the positioning of the balloon catheter 18 within the body of the patient 12. While specific reference is made herein to the balloon catheter 18, it is understood that any suitable type of medical device and/or catheter may be used.

The handle assembly 20 is handled and used by the operator to operate, position and control the balloon catheter 18. The design and specific features of the handle assembly 20 can vary to suit the design requirements of the cryogenic balloon catheter system 10. In the embodiment illustrated in Figure 1, the handle assembly 20 is separate from, but in electrical and/or fluid communication with the control system 14, the fluid source 16 and/or the graphical display 24. In some embodiments, the handle assembly 20 can integrate and/or include at least a portion of the control system 14 within an interior of the handle assembly 20. It is understood that the handle assembly 20 can include fewer or additional components than those specifically illustrated and described herein.

In the embodiment illustrated in Figure 1, the control console 22 includes the control system 14, the fluid source 16 and the graphical display 24. However, in alternative embodiments, the control console 22 can contain additional structures not shown or described herein. Still alternatively, the control console 22 may not include various structures that are illustrated within the control console 22 in Figure 1. For example, in one embodiment, the control console 22 does not include the graphical display 24.

The graphical display 24 provides the operator of the cryogenic balloon catheter system 10 with information that can be used before, during and after the cryoablation procedure. The specifics of the graphical display 24 can vary depending upon the design requirements of the cryogenic balloon catheter system 10, or the specific needs, specifications and/or desires of the operator.

In one embodiment, the graphical display 24 can provide static visual data and/or information to the operator. In addition, or in the alternative, the graphical display 24 can provide dynamic visual data and/or information to the operator, such as video data or any other data that changes over time. Further, in various embodiments, the graphical display 24 can include one or more colors, different sizes, varying brightness, etc., that may act as alerts to the operator. Additionally, or in the alternative, the graphical display can provide audio data or information to the operator.

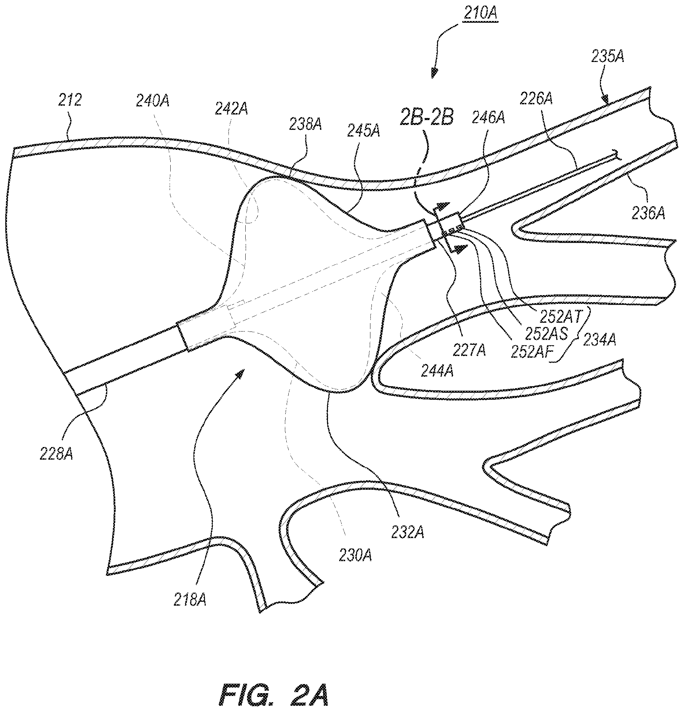

Figure 2A is a simplified side view of a portion of a patient 212 and a portion of one embodiment of the cryogenic balloon catheter system 210A. In this embodiment, the cryogenic balloon catheter system 210A includes a balloon catheter 218A. In the embodiment illustrated in Figure 2A, the balloon catheter 218A includes a guidewire 226A, a guidewire lumen 227A, a catheter shaft 228A, an inner inflatable balloon 230A (sometimes referred to herein as a "first inflatable balloon" or "first balloon"), an outer inflatable balloon 232A (sometimes referred to herein as a "second inflatable balloon" or "second balloon") and a sensor assembly 234A. As used herein, it is recognized that either balloon 230A, 232A can be described as the first balloon or the second balloon. It is also understood that in some embodiments, the balloon catheters described herein may have only one inflatable balloon. In the embodiment illustrated in Figure 2A, the portion of the cryogenic balloon catheter system 210A is positioned within the circulatory system 235A (also sometimes referred to herein as the "body") of the patient 212. The guidewire 226A and guidewire lumen 227A are inserted into a pulmonary vein 236A of the patient, and the catheter shaft 228A and the balloons 230A, 232A are moved along the guidewire 226A and/or guidewire lumen 227A to near an ostium 238A of the pulmonary vein 236A.

In one embodiment, the inner inflatable balloon 230A can be made from a relatively non-compliant or semi-compliant material. Some representative materials suitable for this application include PET (polyethylene terephthalate), nylon, polyurethane, and co-polymers of these materials such as polyether block amide (PEBA), known under its trade name as PEBAX^{®} (supplier Arkema), as non-exclusive examples. In another embodiment, a polyester block copolymer known in the trade as Hytrel^{®} (DuPont^{™}) is also a suitable material for the inner inflatable balloon 230A. The inner inflatable balloon 230A can be relatively inelastic in comparison to the outer inflatable balloon 232A.

In certain embodiments, the outer inflatable balloon 232A can be made from a relatively compliant material. Such materials are well known in the art. One non-exclusive example is aliphatic polyether polyurethanes in which carbon atoms are linked in open chains, including paraffins, olefins, and acetylenes. Another available example goes by the trade name Tecoflex^{®} (Lubrizol). Other available polymers from the polyurethane class of thermoplastic polymers with exceptional elongation characteristics are also suitable for use as the outer inflatable balloon 232A. In one embodiment, either of the balloons 230A, 232A, may be rendered electrically conductive by doping the material from which it is made with a conductive metal or other conductive substance. These electrically conductive balloons are particularly suitable for the outer inflatable balloon 232A described herein.

During use, the inner inflatable balloon 230A can be partially or fully inflated so that at least a portion of the outer surface 240A of the inner inflatable balloon 230A expands against an inner surface 242A of the outer inflatable balloon 232A (although a space is shown between the inner inflatable balloon 230A and the outer inflatable balloon 232A in Figure 2A for clarity and ease in understanding). Further, at certain locations between the inner inflatable balloon 230A and the outer inflatable balloon 232A, there can exist a balloon gap 244A, e.g. an open space between the balloons 230A, 232A, after the inner inflatable balloon 230A is inflated. As provided herein, once the inner inflatable balloon 230A is sufficiently inflated, an outer surface 245A of the outer inflatable balloon 232A can then be positioned within the circulatory system 235A of the patient 212 to abut and/or substantially form a seal with the ostium 238A of the pulmonary vein 236A to be treated.

In the embodiment illustrated in Figure 2A, the sensor assembly 234A is positioned and/or embedded within the guidewire lumen 227A at or near a lumen distal end 246A of the guidewire lumen 227A. As used herein, the lumen distal end 246A is the portion of the guidewire lumen 227A that is first inserted into the circulatory system 235A of the patient 212. In the embodiment illustrated in Figure 2A, the sensor assembly 234A is positioned along the guidewire lumen 227A between a lumen distal end 246A and the outer inflatable balloon 232A. With this design, once the outer inflatable balloon 232A abuts and/or forms a seal with the ostium 238A of the pulmonary vein 236A to be treated, the sensor assembly 234A is positioned within the pulmonary vein 236A to be treated. As such, the sensor assembly 234A can sense various physiological parameters within the pulmonary vein 236A with greater accuracy due to the more stable and/or controlled environment within the sealed-off pulmonary vein 236A.

The sensor assembly 234A is configured to sense one or more physiological parameters within the pulmonary vein 236A. Further, the sensor assembly 234A can provide sensor output regarding the physiological parameters to the control system 14 (illustrated in Figure 1) for storage and/or processing. In one non-exclusive embodiment, the sensor assembly 234A can include a plurality of different sensors, including a first sensor 252AF, a second sensor 252AS, and a third sensor 252AT. However, it is understood that any suitable number of sensors, greater or fewer than three, can alternatively be included in the sensor assembly 234A. Further, in one embodiment, the sensors 252AF, 252AS, 252AT, can include one or more thermocouples.

In one embodiment, the first sensor 252AF, the second sensor 252AS and the third sensor 252AT can include, in no particular order, a pressure sensor, a temperature sensor and an electrode, or any combination thereof. Alternatively, the first sensor 252AF, the second sensor 252AS and the third sensor 252AT can include a plurality of the same type of sensor, and can exclude one or more types of sensors. In one embodiment, the pressure sensor, e.g. a microelectromechanical systems or "MEMS" sensor, can sense the pressure within the blood of the pulmonary vein 236A. The temperature sensor can sense the temperature of the blood within the pulmonary vein 236A. The electrode can sense electrical potentials within the blood of the pulmonary vein 236A. The uses and benefits of these types of sensors during cryogenic ablation procedures are well known and understood.

Additionally, or in the alternative, one of the sensors 252AF, 252AS, 252AT (or an additional sensor within the sensor assembly 234A), can include an ultrasound device/sensor which can assist in determining a location of the guidewire 226A, the guidewire lumen 227A and/or the sensor assembly 234A within the circulatory system of the patient 212. More specifically, the ultrasound device/sensor can provide a sensor output that accurately shows a user of the cryogenic balloon catheter system 210A the location of the sensor assembly 234A within the pulmonary vein 236A while the cryogenic balloon catheter system 210A is in use.

In certain embodiments, the control system 14 (illustrated in Figure 1) is configured to process and integrate the sensor output to determine proper functioning of the cryogenic balloon catheter system 210A. Based on the sensor output, the control system 14 can determine that certain modifications to the functioning of the cryogenic balloon catheter system 210A are required.

The control system 14 can abort the delivery of cryogenic fluid 26 (illustrated in Figure 1), can increase the fluid flow rate to get more cooling, reduce the fluid flow rate, and/or can have an initial flowrate to reduce temperature to a set point then change the flow rate to maintain a set temperature. In certain embodiments, the control system 14 can also change the cycle time and/or amount of fluid delivery.

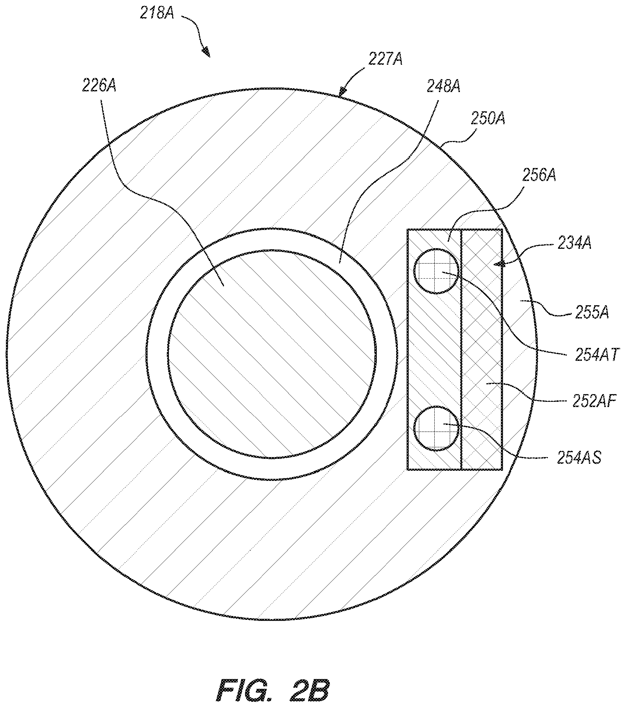

Figure 2B is a cross-sectional view of the balloon catheter 218A taken on line 2B-2B in Figure 2A. In this embodiment, the balloon catheter 218A includes the guidewire 226A, the guidewire lumen 227A, and the sensor assembly 234A. In this embodiment, the guidewire lumen 227A has a lumen interior 248A and an outer perimeter 250A. The guidewire 226A is positioned within the lumen interior 248A of the guidewire lumen 227A. In this embodiment, the sensor assembly 234A is positioned between the lumen interior 248A and the outer perimeter 250A of the guidewire lumen 227A.

As shown in Figure 2B, the sensor assembly 234A includes the first sensor 252AF. Sensors 252AS, 252AT, are not shown in Figure 2B. However, the sensor assembly 234A includes sensor conductors 254AS, 254AT, which transmit the sensor output for sensors 252AS, 252AT (illustrated in Figure 2A) to the control system 14 (illustrated in Figure 1). The sensor assembly 234A also includes a sensor conductor (not shown) for the first sensor 252AF in order to transmit sensor output for sensor 252AF to the control system 14. Alternatively, the sensors 252AF, 252AS, 252AT, can communicate wirelessly with the control system 14.

In one embodiment, the sensor assembly 234A can be at least partially, if not fully, covered by a sensor outer cover 255A. The sensor outer cover 255A can include an elastomeric material that isolates one or more of the sensors 252AF, 252AS, 252AT, from the blood in the circulatory system of the patient 212, and can inhibit damage to one or more of the sensors 252AF, 252AS, 252AT, during insertion and removal from the patient 212. In one embodiment, the sensor outer cover 255A can be part of the guidewire lumen 227A. The sensor assembly 234A can also be housed within a sensor housing 256A that can form part of the guidewire lumen 227A.

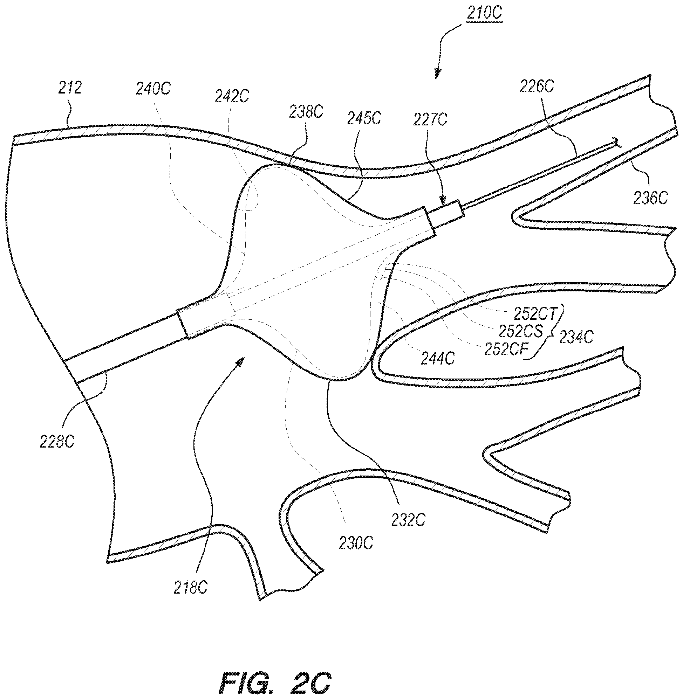

Figure 2C is a simplified side view of a portion of a patient 212 and a portion of another embodiment of the cryogenic balloon catheter system 210C. In this embodiment, the cryogenic balloon catheter system 210C includes a balloon catheter 218C. In the embodiment illustrated in Figure 2A, the balloon catheter 218C includes a guidewire 226C, a guidewire lumen 227C, a catheter shaft 228C, an inner inflatable balloon 230C, an outer inflatable balloon 232C and a sensor assembly 234C. In this embodiment, the portion of the cryogenic balloon catheter system 210C illustrated in Figure 2C is positioned within the circulatory system of the patient 212. The guidewire 226C and guidewire lumen 227C are inserted into a pulmonary vein 236C of the patient 212, and the catheter shaft 228C and the balloons 230C, 232C are moved along the guidewire 226C and/or the guidewire lumen 227C to near an ostium 238C of the pulmonary vein 236C.

The inner inflatable balloon 230C and the outer inflatable balloon 232C can be constructed from materials in a somewhat similar manner as those previously described herein. Further, the inner inflatable balloon 230C and the outer inflatable balloon 232C can operate in a somewhat similar manner as previously described herein. However, in the embodiment illustrated in Figure 2C, the sensor assembly 234C is positioned between the inner inflatable balloon 230C and the outer inflatable balloon 232C. In one embodiment, the sensor assembly 234C can be adhered or otherwise secured to an outer surface 240C of the inner inflatable balloon 230C. In certain embodiments, the sensor assembly 234C can be adhered or otherwise secured to the inner inflatable balloon 230C in the balloon gap 244C.

Alternatively, the sensor assembly 234C can be positioned in another location between the inner inflatable balloon 230C and the outer inflatable balloon 232C. As such, the sensor assembly 234C can sense various physiological parameters that are occurring at or near the ostium 238C of the pulmonary vein 236C. One advantage to placing the sensor assembly 234C between the two balloons 230C, 232C, includes providing a robust bond to an outer surface 240C of the inner inflatable balloon 230C, which is relatively non-compliant, thereby providing a better surface for bonding structures such as the sensor assembly 234C described herein. In contrast, bonding structures to a more compliant outer inflatable balloon 232C can cause wires and/or sensors to become loose, which can cause entangling with delicate cardiac structures such as valves, etc. By bonding to the outer surface 240C of the inner inflatable balloon 230C, a safety benefit is thereby achieved.

In this embodiment, the sensor assembly 234C is configured to sense one or more physiological parameters near or within the pulmonary vein 236C. Further, the sensor assembly 234C can provide sensor output to the control system 14 (illustrated in Figure 1) for storage and/or processing regarding the physiological parameters. In one non-exclusive embodiment, the sensor assembly 234C can include a plurality of different sensors, including a first sensor 252CF, a second sensor 252CS, and a third sensor 252CT. However, it is understood that any suitable number of sensors can be included in the sensor assembly 234C, which may be fewer or greater than three sensors. In one embodiment, the sensor assembly 234C can include a flex circuit that can be bonded to the outer surface 240C of the inner inflatable balloon 230C. Alternatively, the sensor assembly 234C can include any other suitable type of electrical communication device or wiring between the sensors 252CF, 252CS, 252CT, and the control system 14. Still alternatively, the sensors 252CF, 252CS, 252CT, can communicate wirelessly with the control system 14.

The sensors 252CF, 252CS, 252CT, can operate in a somewhat similar manner as those previously described herein. In certain embodiments, the control system 14 is configured to process and integrate the sensor output to determine proper functioning of the cryogenic balloon catheter system 210C. Based on the sensor output, the control system 14 can determine that certain modifications to the functioning of the cryogenic balloon catheter system 210C are required.

Although the foregoing embodiments show and describe various sensors being positioned either (1) between the lumen distal end 246A and the outer inflatable balloon 232A, or (2) between the inner inflatable balloon 230C and the outer inflatable balloon 232C, it is recognized that an alternative embodiment can include one or more sensors being positioned between the lumen distal end 246A and the outer inflatable balloon 232A, and one or more sensors being positioned between the inner inflatable balloon 230C and the outer inflatable balloon 232C. In other words, sensors can be positioned in both locations in this alternative embodiment without deviating from the spirit of the cryogenic balloon catheter system 10 described herein. Additionally, one or more of the sensors can be positioned on the guidewire 226A. All of the data collected from the sensors, regardless of the position of the sensors, can be sent to the control system 14 for use by a user (health care physician or other user) or by the control system 14 itself.

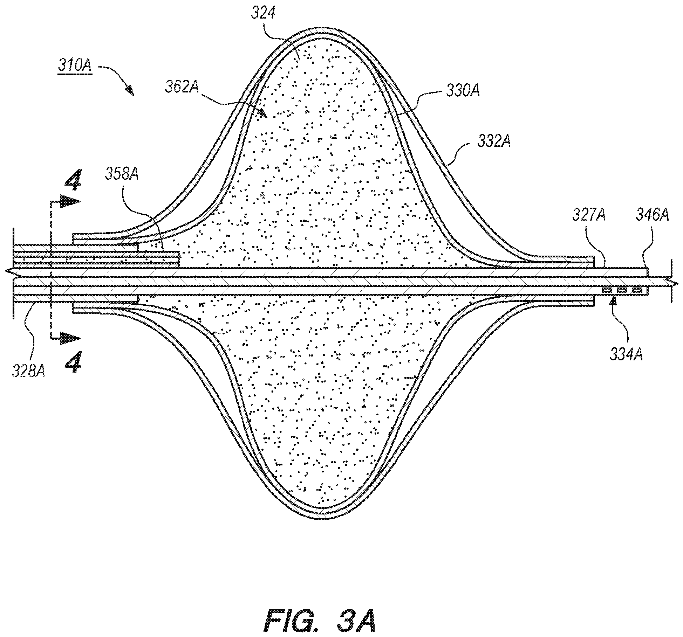

Figure 3A is a cross-sectional view of one embodiment of a portion of the cryogenic balloon catheter system 310A. In this embodiment, the cryogenic balloon catheter system 310A includes a guidewire lumen 327A, a catheter shaft 328A, an inner inflatable balloon 330A, an outer inflatable balloon 332A, a sensor assembly 334A and a fluid injection line 358A. In the embodiment illustrated in Figure 3A, the sensor assembly 334A is positioned and/or embedded outside of the outer inflatable balloon 332A within the catheter shaft 328A at or near a shaft distal end 346A of the catheter shaft 328A, as described previously herein. Further, in this embodiment, the fluid injection line 358A extends through the outer inflatable balloon 332A and the inner inflatable balloon 330A, and into an inner inflatable balloon interior 362A. The fluid injection line 358A can be configured and/or positioned in any suitable manner. Although the fluid injection line 358A is illustrated in Figure 3A as a straight tube, the fluid injection line 358A can be coiled, or can have any other suitable geometry or configuration. The control system 14 (illustrated in Figure 1) can direct dispensing of the cryogenic fluid 324 into the inner inflatable balloon interior 362A to appropriately fill the inner inflatable balloon 330A during an ablation procedure.

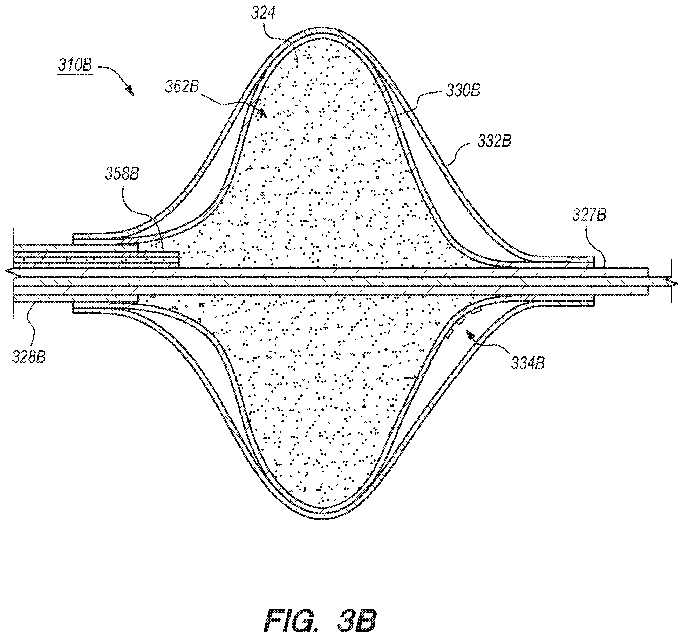

Figure 3B is a cross-sectional view of another embodiment of a portion of the cryogenic balloon catheter system 310B. In this embodiment, the cryogenic balloon catheter system 310B includes a guidewire lumen 327B, a catheter shaft 328B, an inner inflatable balloon 330B, an outer inflatable balloon 332B, a sensor assembly 334B and a fluid injection line 358B. In the embodiment illustrated in Figure 3B, the sensor assembly 334B is positioned between the inner inflatable balloon 330B and the outer inflatable balloon 332B, as described previously herein. Further, in this embodiment, the fluid injection line 358B extends through the outer inflatable balloon 332B and the inner inflatable balloon 330B, and into an inner inflatable balloon interior 362B. The fluid injection line 358B can be configured and/or positioned in any suitable manner. Although the fluid injection line 358B is illustrated in Figure 3B as a straight tube, the fluid injection line 358B can be coiled, or can have any other suitable geometry or configuration. The control system 14 (illustrated in Figure 1) can direct dispensing of the cryogenic fluid 324 into the inner inflatable balloon interior 362B to appropriately fill the inner inflatable balloon 330B during an ablation procedure.

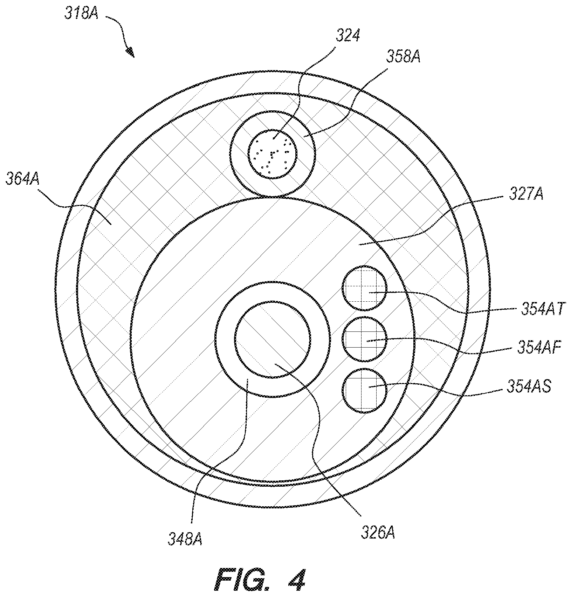

Figure 4 is a cross-sectional view of a portion of the cryogenic balloon catheter system 310A including the balloon catheter 318A taken on line 4-4 in Figure 3A. In the embodiment illustrated in Figure 4, the catheter shaft 328A encircles the guidewire lumen 327A and the fluid injection line 358A. Additionally, within the guidewire lumen 327A are the sensor conductors 354AF, 354AS, 354AT, the lumen interior 348A, the guidewire 326A and a catheter housing 364A. The catheter housing 364A houses the various structures within the catheter shaft 328A.

An alternative embodiment includes placing a pressure sensor into an assembly comprised of three conductors, a sensor housing, and a sealed tube enclosing the wiring. In this embodiment, the assembly is routed internally through the catheter, from the shaft distal end of the catheter shaft to the handle assembly and/or the control system.

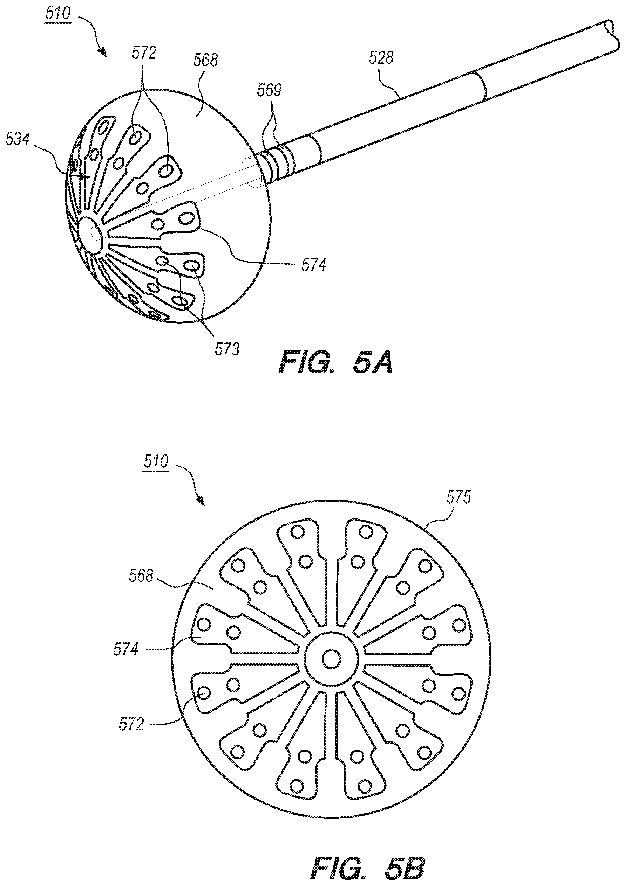

Figure 5A is a perspective view of a portion of another embodiment of the cryogenic balloon catheter system 510. In this embodiment, the cryogenic balloon catheter system 510 includes a catheter shaft 528, an inflatable balloon 568 and a portion of a sensor assembly 534. In the embodiment illustrated in Figure 5A, the inflatable balloon 568 is shown in an inflated state. Further, in Figure 5A, the guidewire 226A, 226C (illustrated in Figures 2A and 2C, respectively), has been omitted for clarity.

In the embodiment illustrated in Figure 5A, the catheter shaft 528 can include one or more reference electrodes 569 (two reference electrodes 569 forming a thermocouple are illustrated in Figure 5A). The reference electrodes 569 can be used to provide a reference sensor output to provide the operator with a known temperature at or near their location. As provided in greater detail herein, the reference sensor output of the reference electrodes 569 can be compared to sensor output of other portions of the sensor assembly 534 to aid in determining positioning (mapping) of the inflatable balloon 568, temperature, pressure, etc., or for any suitable purpose during an ablation procedure.

In this embodiment, the inflatable balloon 568 can represent either the inner inflatable balloon 230A (illustrated in Figure 2A) or the outer inflatable balloon 232A (illustrated in Figure 2A). Alternatively, the inflatable balloon 568 can be a single inflatable balloon with no balloon positioned within its interior space.

The sensor assembly 534 can include a plurality of electrodes 572 that are secured to the inflatable balloon 568 on an outer surface 240A (illustrated in Figure 2A) of the inner inflatable balloon 230A (illustrated in Figure 2A), on an inner surface 242A (illustrated in Figure 2A) of the outer inflatable balloon 232A (illustrated in Figure 2A), and/or on an outer surface 245A (illustrated in Figure 2A) of the outer inflatable balloon 232A. In one embodiment, the electrodes 572 can be adhered to the inflatable balloon 568 with a flexible adhesive. Alternatively, the electrodes 572 can be secured to the inflatable balloon 568 by any other suitable manner. Any suitable number of electrodes 572 can be used.

In the embodiment illustrated in Figure 5A, the electrodes 572 can be in electrode pairs 573 (one pair of electrodes 572 that form a thermocouple are identified in Figure 5A), with each electrode pair 573 positioned on and/or embedded within a flex circuit 574. In this embodiment, each electrode pair 573 can provide sensor output that is compared to the sensor output of the reference electrodes 569, or any other electrode pair(s), in order to determine the temperature at or near any particular electrode pair 573. In the embodiment illustrated in Figure 5A, a plurality of flex circuits 574 are positioned on and/or secured to the inflatable balloon 568.

Figure 5B is an end view of a portion of the cryogenic balloon catheter system 510 illustrated in Figure 5A, shown in the inflated state. In Figure 5B, the guidewire 226A, 226C (illustrated in Figures 2A and 2C, respectively), has been omitted for clarity. In this embodiment, the flex circuits 574 and/or the electrodes 572 can be positioned in a somewhat radial or spoke-like pattern on a surface of the inflatable balloon 568. For example, in one embodiment, the flex circuits 574 and/or the electrodes 572 can extend in a substantially evenly spaced, radial pattern on the inflatable balloon 568. Alternatively, the flex circuits 574 and/or the electrodes 572 can have any other suitable configuration on the inflatable balloon 568. In certain embodiments, in the radial configuration (as well as other configurations), the electrodes 572 can provide mapping information to the operator so that the operator can determine the positioning of the inflatable balloon 568 within the body of the patient 12 (illustrated in Figure 1, for example).

In the embodiment illustrated in Figure 5B, there are twelve flex circuits 574 positioned on the inflatable balloon 568, each with two electrodes 572. It is recognized, however, that any suitable number (greater or fewer than twelve) or combination of flex circuits 574 and/or electrodes 572 can be used. In the embodiment illustrated in Figures 5A and 5B, the electrodes 572 and/or the flex circuits 574 are positioned distal to a maximum circumference 575 of the inflatable balloon 568, and are not positioned along or about the maximum circumference 575 of the inflatable balloon 568. As used herein, the "maximum circumference" is the largest circumference of the inflatable balloon 568 while the inflatable balloon 568 is in the inflated state. When in the deflated state, the inflatable balloon 568 is less cumbersome and more easily positioned and/or withdrawn into a sheath (not shown) that surrounds the inflatable balloon 568 during insertion and/or removal of the balloon catheter 18 (illustrated in Figure 1) into or from the patient 12. In an alternative embodiment, the electrodes 572 and/or the flex circuits 574 are positioned proximal to the maximum circumference 575 of the inflatable balloon 568, and are not positioned along or about the maximum circumference 575 of the inflatable balloon 568.

In one embodiment, the flex circuits 574 are in electrical communication with the control system 14 (illustrated in Figure 1) via sensor conductors 254AS, 254AT, 354AF, 354AS, 354AT (illustrated in Figures 2B and/or 4), which can be substantially similar and somewhat similarly positioned as those previously described herein. Alternatively, the sensor conductors can be positioned in another suitable manner. For example, in one embodiment, one or more of the sensor conductors can extend along a portion of the inflatable balloon 568, and can enter or extend along the guidewire lumen 227A (illustrated in Figure 2A), 227C (illustrated in Figure 2C), at the shaft distal end 346A (illustrated in Figure 3A, for example).

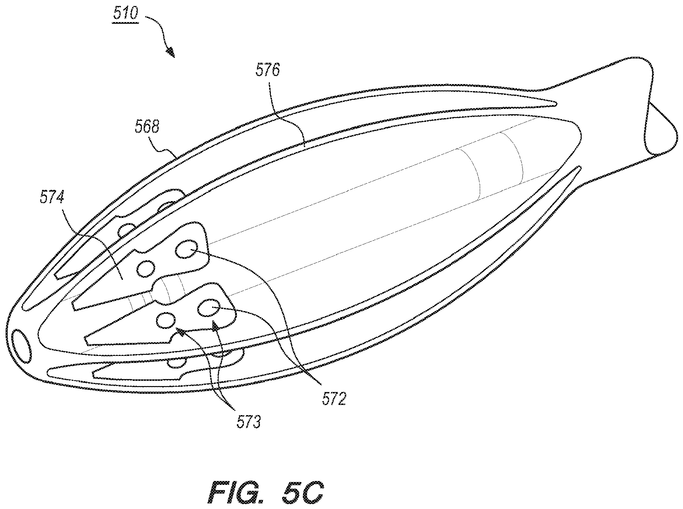

Figure 5C is a perspective view of a portion of the cryogenic balloon catheter system 510 illustrated in Figure 5A, with the inflatable balloon 568 shown in a substantially deflated state. In this embodiment, the inflatable balloon 568 can be either the inner inflatable balloon or the outer inflatable balloon. Alternatively, the inflatable balloon 568 can be a single balloon rather than one of two balloons as previously described herein.

In the deflated state, the inflatable balloon 568 becomes somewhat pleated to permit spines 576 between one or more of the flex circuits 574, which each contains at least one electrode pair 573 of electrodes 572. With this design, the flex circuits 574 themselves are not folded when the inflatable balloon 568 is in the deflated state. However, spines 576 between the flex circuits 574 inhibit unwanted peeling off of the flex circuits 574 from the inflatable balloon 568 during deflation and while in the deflated state. Further, in the deflated state, the inflatable balloon 568 is pleated which facilitates a smaller, more organized profile of the inflatable balloon 568 for removal from the body of the patient 12 (illustrated in Figure 1).

In one embodiment, in the deflated state, two flex circuits 574 are positioned between two adjacent spines 576. In one embodiment, the two flex circuits 574 that are positioned between two adjacent spines 576 are adjacent to one another. Alternatively, the two flex circuits 574 that are positioned between two adjacent spines 576 need not be adjacent to one another. With this design, two such adjacent flex circuits 574 and/or two adjacent electrode pairs 573 will substantially face one another upon deflation of the inflatable balloon 568. Stated another way, in one embodiment, the inflatable balloon 568 will include a pleat or crease between two such adjacent flex circuits 574 and/or the two such adjacent electrode pairs 573, such that each two such adjacent flex circuits 574 will substantially face one another when the inflatable balloon 568 is substantially and/or completely deflated.

In this embodiment, the flex circuits 574 will alternate substantially facing an adjacent flex circuit 574 on one side, and facing substantially away from the other adjacent flex circuit 574 on an opposite side of the spine 576 when the inflatable balloon 568 is substantially completely deflated. In such embodiment, two flex circuits 574 are positioned between two adjacent spines 576 of the inflatable balloon 568. In other words, as the spines 576 are moved closer toward one another when the inflatable balloon 568 is in the deflated state, the two flex circuits 574 that are positioned between two adjacent spines 576 can rotate toward one another so that the flex circuits 574 substantially face one another. Further, with this design, folding or creasing of the flex circuits 574 is inhibited.

It is understood that although a number of different embodiments of the cryogenic balloon catheter system 10 have been illustrated and described herein, one or more features of any one embodiment can be combined with one or more features of one or more of the other embodiments, provided that such combination satisfies the intent of the present invention.

While a number of exemplary aspects and embodiments of a cryogenic balloon catheter system 10 have been discussed above, those of skill in the art will recognize certain modifications, permutations, additions and sub-combinations thereof. It is therefore intended that the scope of the present invention is defined by the appended claims.

## Claims

1. An intravascular catheter system (10; 510) for use in a cryoablation procedure for treating a condition in a body, the intravascular catheter system comprising:
a catheter shaft (228A; 528) having a shaft distal end that is configured to be selectively positioned within the body;
a first inflatable balloon (230A; 232A; 568) positioned near the distal end of the catheter shaft (228A; 528), the first inflatable balloon (230A; 232A; 568) being configured to move between an inflated state and a substantially deflated state, the first inflatable balloon (230A; 232A; 568) having a maximum circumference in the inflated state;
a plurality of electrodes (572) that are attached to the first inflatable balloon (230A; 232A; 568), the plurality of electrodes (572) configured to sense a physiological parameter within the body, the plurality of electrodes (572) being positioned away from the maximum circumference of the first inflatable balloon (230A; 232A; 568) so that none of the electrodes (572) are positioned on the maximum circumference of the first inflatable balloon (230A; 232A; 568); and
a plurality of flex circuits (574) secured to the first inflatable balloon (230A; 232A; 568), wherein the electrodes (572) are coupled to the first inflatable balloon (230A; 232A; 568) via the plurality of flex circuits (574), and wherein at least two electrodes are positioned on each of the flex circuits (574) , and wherein each of the flex circuits (574) is positioned distal to the maximum circumference the first inflatable balloon (230A; 232A; 568);
a controller and a plurality of conductors, each conductor carrying an electrical signal between at least one of the electrodes and the controller, the electrical signal being based on the physiological parameter; and
a guidewire lumen (227A) and a guidewire (226A) that is positioned at least partially within the guidewire lumen (227A), wherein the first inflatable balloon (230A; 232A; 568) is attached to the guidewire lumen (227A), wherein the guidewire lumen (227A) has a lumen distal end (246A), and wherein each conductor is routed from at least one of the electrodes to the controller via the lumen distal end (246A) of the guidewire lumen (227A).

2. The intravascular catheter system (10; 510) of claim 1, wherein the first inflatable balloon (230A; 232A; 568) has an inner surface and an opposed outer surface, and wherein the electrodes (572) are positioned on the inner surface of the first inflatable balloon (230A; 232A; 568).

3. The intravascular catheter system (10; 510) of claim 1, wherein the first inflatable balloon (230A; 232A; 568) has an inner surface and an opposed outer surface, and wherein the electrodes are positioned on the outer surface of the first inflatable balloon (230A; 232A; 568).

4. The intravascular catheter system (10; 510) of any of claims 2-3, wherein two of the electrodes on each flex circuit (574) form a thermocouple.

5. The intravascular catheter system (10; 510) of any of claims 1-4, wherein at least eight flex circuits (574) are positioned on the first inflatable balloon (230A; 232A; 568).

6. The intravascular catheter system (10; 510) of any of claims 1-5, wherein at least twelve flex circuits (574) are positioned on the first inflatable balloon (230A; 232A; 568).

7. The intravascular catheter system (10; 510) of any of claims 1-6, wherein the inflatable balloon (230A; 568) includes a plurality of spines when the first inflatable balloon is in the substantially deflated state.

8. The intravascular catheter system (10; 510) of claim 7, wherein two flex circuits (574) are positioned between two adjacent spines.

9. The intravascular catheter system (10; 510) of claim 8, wherein the two flex circuits (574) substantially face one another when the first inflatable balloon (230A; 232A; 568) is in the substantially deflated state.

10. The intravascular catheter system (10; 510) of any of claims 1-9, further comprising a second inflatable balloon (232A) that is positioned within the first inflatable balloon (230A).

11. The intravascular catheter system (10; 510) of claim 1 further comprising a pair of reference electrodes that are positioned away from the first inflatable balloon (230A; 232A; 568), the pair of reference electrodes forming a thermocouple that is configured to sense a temperature of a portion of the body, wherein the reference electrodes are configured to generate a reference sensor output, and wherein two of the plurality of electrodes are configured to generate a sensor output that is compared to the reference sensor output to determine a temperature of a portion of the body.

## Patentansprüche

1. Intravaskuläres Kathetersystem (10; 510) zur Verwendung in einem Kryoablationsverfahren zur Behandlung einer Gegebenheit in einem Körper, wobei das intravaskuläre Kathetersystem umfasst:
einen Katheterschaft (228A; 528) mit einem distalen Schaftende, das für eine selektive Positionierung innerhalb des Körpers ausgebildet ist;
einen ersten aufpumpbaren Ballon (230A; 232A; 568), der in der Nähe des distalen Endes des Katheterschafts (228A; 528) positioniert ist, wobei der erste aufpumpbare Ballon (230A; 232A; 568) für eine Bewegung zwischen einem aufgepumpten Zustand und einem im Wesentlichen schlaffen Zustand ausgebildet ist, wobei der erste aufpumpbare Ballon (230A; 232A; 568) im aufgepumpten Zustand einen maximalen Umfang aufweist;
eine Mehrzahl von Elektroden (572), die an dem ersten aufpumpbaren Ballon (230A; 232A; 568) angebracht sind, wobei die Mehrzahl von Elektroden (572) ausgebildet ist, um einen physiologischen Parameter innerhalb des Körpers zu erfassen, wobei die Mehrzahl von Elektroden (572) abseits von dem maximalen Umfang des ersten aufpumpbaren Ballons (230A; 232A; 568) positioniert ist, so dass keine von den Elektroden (572) an dem maximalen Umfang des ersten aufpumpbaren Ballons (230A; 232A; 568) positioniert ist; und
eine Mehrzahl von flexiblen Schaltungen (574), die an dem ersten aufpumpbaren Ballon (230A; 232A; 568) befestigt sind, wobei die Elektroden (572) über die Mehrzahl von flexiblen Schaltungen (574) mit dem ersten aufpumpbaren Ballon (230A; 232A; 568) gekoppelt sind und wobei mindestens zwei Elektroden an jeder von den flexiblen Schaltungen (574) positioniert sind, und wobei jede von den flexiblen Schaltungen (574) distal zu dem maximalen Umfang des ersten aufpumpbaren Ballons (230A; 232A; 568) positioniert ist;
einen Controller und eine Mehrzahl von Leitern, wobei jeder Leiter ein elektrisches Signal zwischen mindestens einer von den Elektroden und dem Controller trägt, wobei das elektrische Signal auf dem physiologischen Parameter basiert; und
ein Führungsdrahtlumen (227A) und einen Führungsdraht (226A), der zumindest zum Teil innerhalb des Führungsdrahtlumens (227A) positioniert ist, wobei der erste aufpumpbare Ballon (230A; 232A; 568) an dem Führungsdrahtlumen (227A) angebracht ist, wobei das Führungsdrahtlumen (227A) ein distales Lumenende (246A) aufweist und wobei jeder Leiter von mindestens einer von den Elektroden aus über das distale Lumenende (246A) zum Führungsdrahtlumen (227A) geführt ist.

2. Intravaskuläres Kathetersystem (10; 510) nach Anspruch 1, wobei der erste aufpumpbare Ballon (230A; 232A; 568) eine Innenfläche und eine dazu entgegengesetzte Außenfläche aufweist, und wobei die Elektroden (572) an der Innenfläche des ersten aufpumpbaren Ballons (230A; 232A; 568) positioniert sind.

3. Intravaskuläres Kathetersystem (10; 510) nach Anspruch 1, wobei der erste aufpumpbare Ballon (230A; 232A; 568) eine Innenfläche und eine dazu entgegengesetzte Außenfläche aufweist, und wobei die Elektroden an der Außenfläche des ersten aufpumpbaren Ballons (230A; 232A; 568) positioniert sind.

4. Intravaskuläres Kathetersystem (10; 510) nach einem der Ansprüche 2-3, wobei zwei von den Elektroden an jeder flexiblen Schaltung (574) ein Thermoelement bilden.

5. Intravaskuläres Kathetersystem (10; 510) nach einem der Ansprüche 1-4, wobei mindestens acht flexible Schaltungen (574) an dem ersten aufpumpbaren Ballon (230A; 232A; 568) positioniert sind.

6. Intravaskuläres Kathetersystem (10; 510) nach einem der Ansprüche 1-5, wobei mindestens zwölf flexible Schaltungen (574) an dem ersten aufpumpbaren Ballon (230A; 232A; 568) positioniert sind.

7. Intravaskuläres Kathetersystem (10; 510) nach einem der Ansprüche 1-6, wobei der aufpumpbare Ballon (230A; 568) eine Mehrzahl von Graten aufweist, wenn der erste aufpumpbare Ballon den im Wesentlichen schlaffen Zustand aufweist.

8. Intravaskuläres Kathetersystem (10; 510) nach Anspruch 7, wobei zwei flexible Schaltungen (574) zwischen zwei einander benachbarten Graten positioniert sind.

9. Intravaskuläres Kathetersystem (10; 510) nach Anspruch 8, wobei die zwei flexiblen Schaltungen (574) einander im Wesentlichen gegenüberliegen, wenn der erste aufpumpbare Ballon (230A; 232A; 568) den im Wesentlichen schlaffen Zustand aufweist.

10. Intravaskuläres Kathetersystem (10; 510) nach einem der Ansprüche 1-9, ferner einen zweiten aufpumpbaren Ballon (232A) umfassend, der innerhalb des ersten aufpumpbaren Ballons (230A) positioniert ist.

11. Intravaskuläres Kathetersystem (10; 510) nach Anspruch 1, ferner ein Paar von Bezugselektroden umfassend, die abseits von dem ersten aufpumpbaren Ballon (230A; 232A; 568) positioniert sind, wobei das Paar von Bezugselektroden ein Thermoelement bildet, das ausgebildet ist, um eine Temperatur eines Teils des Körpers zu erfassen, wobei die Bezugselektroden ausgebildet sind, um eine Bezugssensorausgabe zu erzeugen, und wobei zwei von der Mehrzahl von Elektroden ausgebildet sind, um eine Sensorausgabe zu erzeugen, die mit der Bezugssensorausgabe verglichen wird, um eine Temperatur eines Teils des Körpers zu bestimmen.

## Revendications

1. Système de cathéter intravasculaire (10 ; 510) destiné à être utilisé lors d'une procédure de cryoablation pour traiter une condition dans un corps, le système de cathéter intravasculaire comprenant :
un arbre de cathéter (228A ; 528) qui comporte une extrémité distale d'arbre qui est configurée pour être positionnée de façon sélective à l'intérieur du corps ;
un premier ballonnet gonflable (230A; 232A ; 568) qui est positionné à proximité de l'extrémité distale de l'arbre de cathéter (228A ; 528), le premier ballonnet gonflable (230A ; 232A ; 568) étant configuré pour être déplacé entre un état gonflé et un état sensiblement dégonflé, le premier ballonnet gonflable (230A ; 232A ; 568) présentant une circonférence maximum dans l'état gonflé ;
une pluralité d'électrodes (572) qui sont liées au premier ballonnet gonflable (230A ; 232A ; 568), les électrodes de la pluralité d'électrodes (572) étant configurées pour détecter un paramètre physiologique à l'intérieur du corps, la pluralité d'électrodes (572) étant positionnée à distance de la circonférence maximum du premier ballonnet gonflable (230A ; 232A ; 568) de telle sorte qu'aucune des électrodes (572) ne soit positionnée sur la circonférence maximum du premier ballonnet gonflable (230A ; 232A ; 568) ; et
une pluralité de circuits imprimés souples (574) qui sont fixés au premier ballonnet gonflable (230A ; 232A ; 568), dans lequel les électrodes (572) sont couplées au premier ballonnet gonflable (230A ; 232A ; 568) par l'intermédiaire de la pluralité de circuits imprimés souples (574), et dans lequel au moins deux électrodes sont positionnées sur chacun des circuits imprimés souples (574), et dans lequel chacun des circuits imprimés souples (574) est positionné de façon distale par rapport à la circonférence maximum du premier ballonnet gonflable (230A ; 232A ; 568) ;
un contrôleur et une pluralité de conducteurs, chaque conducteur acheminant un signal électrique entre au moins l'une des électrodes et le contrôleur, le signal électrique étant basé sur le paramètre physiologique ; et
une lumière de fil de guidage (227A) et un fil de guidage (226A) qui est positionné au moins partiellement à l'intérieur de la lumière de fil de guidage (227A), dans lequel le premier ballonnet gonflable (230A; 232A ; 568) est lié à la lumière de fil de guidage (227A), dans lequel la lumière de fil de guidage (227A) comporte une extrémité distale de lumière (246A) et dans lequel chaque conducteur chemine depuis au moins l'une des électrodes jusqu'au contrôleur via l'extrémité distale de lumière (246A) de la lumière de fil de guidage (227A).

2. Système de cathéter intravasculaire (10 ; 510) selon la revendication 1, dans lequel le premier ballonnet gonflable (230A; 232A ; 568) comporte une surface interne et une surface externe opposée, et dans lequel les électrodes (572) sont positionnées sur la surface interne du premier ballonnet gonflable (230A; 232A ; 568).

3. Système de cathéter intravasculaire (10 ; 510) selon la revendication 1, dans lequel le premier ballonnet gonflable (230A; 232A ; 568) comporte une surface interne et une surface externe opposée, et dans lequel les électrodes sont positionnées sur la surface externe du premier ballonnet gonflable (230A ; 232A ; 568).

4. Système de cathéter intravasculaire (10 ; 510) selon l'une quelconque des revendications 2 et 3, dans lequel deux des électrodes sur chaque circuit imprimé souple (574) forment un thermocouple.

5. Système de cathéter intravasculaire (10 ; 510) selon l'une quelconque des revendications 1 à 4, dans lequel au moins huit circuits imprimés souples (574) sont positionnés sur le premier ballonnet gonflable (230A ; 232A ; 568).

6. Système de cathéter intravasculaire (10 ; 510) selon l'une quelconque des revendications 1 à 5, dans lequel au moins douze circuits imprimés souples (574) sont positionnés sur le premier ballonnet gonflable (230A ; 232A ; 568).

7. Système de cathéter intravasculaire (10 ; 510) selon l'une quelconque des revendications 1 à 6, dans lequel le ballonnet gonflable (230A ; 568) inclut une pluralité de nervures de structuration lorsque le premier ballonnet gonflable est dans l'état sensiblement dégonflé.

8. Système de cathéter intravasculaire (10 ; 510) selon la revendication 7, dans lequel deux circuits imprimés souples (574) sont positionnés entre deux nervures de structuration adjacentes.

9. Système de cathéter intravasculaire (10 ; 510) selon la revendication 8, dans lequel les deux circuits imprimés souples (574) se font sensiblement mutuellement face lorsque le premier ballonnet gonflable (230A ; 232A ; 568) est dans l'état sensiblement dégonflé.

10. Système de cathéter intravasculaire (10 ; 510) selon l'une quelconque des revendications 1 à 9, comprenant en outre un second ballonnet gonflable (232A) qui est positionné à l'intérieur du premier ballonnet gonflable (230A).

11. Système de cathéter intravasculaire (10 ; 510) selon la revendication 1, comprenant en outre une paire d'électrodes de référence qui sont positionnées à distance du premier ballonnet gonflable (230A ; 232A ; 568), la paire d'électrodes de référence formant un thermocouple qui est configuré pour détecter une température d'une partie du corps, dans lequel les électrodes de référence sont configurées pour générer une sortie de capteur de référence, et dans lequel deux de la pluralité d'électrodes sont configurées pour générer une sortie de capteur qui est comparée à la sortie de capteur de référence pour déterminer une température d'une partie du corps.
